# EUROPEAN PATENT APPLICATION

(11) **EP 2 412 364 A1**
(43) Date of publication of application: **01.02.2012**
(21) Application number: 10755848.8
(22) Date of filing: 09.03.2010
(51) Int. Cl.: A61K 8/891, A61K 8/04, A61K 8/39, A61K 8/86, A61Q 19/00, C08K 5/06, C08L 71/02, C08L 83/04, C08L 83/06

(54) **SOLUBILIZED COMPOSITION**

(30) Priority: 27.03.2009 JP 2009080260
(71) Applicant: Shiseido Company, Ltd., Chuo-ku Tokyo 104-8010 (JP)
(72) Inventor: TESHIGAWARA Takashi, Yokohama-shi Kanagawa 224-8558 (JP); WATANABE Kei, Yokohama-shi Kanagawa 224-8558 (JP); ARAKI Hidefumi, Yokohama-shi Kanagawa 224-8558 (JP)
(74) Representative: Lippert, Stachow & Partner
(86) International application number: PCT/JP2010/053856
(87) International publication number: WO 2010/110047

(57) **Abstract**

This invention provides a solubilized composition in which a carboxy-modified silicone is solubilized in a large amount and stably. The solubilized composition is **characterized by** consisting of (a) one or more nonionic surfactant(s), having a weighted average of the HLB value of 10 to 15, selected from polyoxyethylene alkyl ether type nonionic surfactant represented by the following formula (I) or (II):

R¹-O-(CH₂CH₂O)ₐ-H (I)

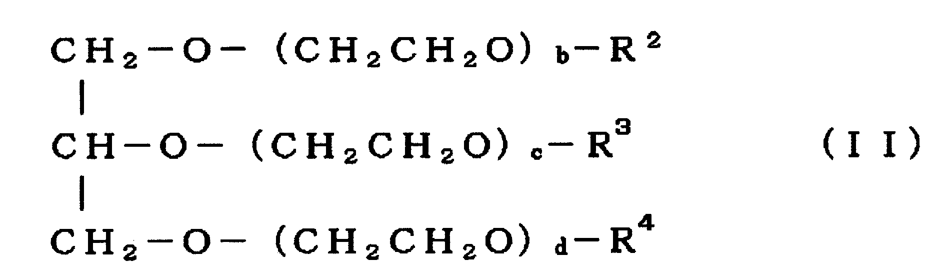

wherein R¹ represents a branched chain alkyl group having 8 to 18 carbon atoms; a represents an integer satisfying 3 ≤a ≤ 40; any one or more of R², R³ and R⁴ are each a branched chain alkyl group having 8 to 18 carbon atoms and the other(s) is a hydroxy group; and b, c and d each represent an integer satisfying 3 ≤ b + c + d ≤ 40,
(b) a carboxy-modified silicone, and
(c) water, and

wherein the amount of (b) the carboxy-modified silicone is three times or less the weight of (a) the nonionic surfactant(s).

## Description

### RELATED APPLICATIONS

This application claims the priority of Japanese Patent Application No. 2009-080260 filed on March 27, 2009, which are incorporated herein by reference.

### FIELD OF THE INVENTION

The present invention relates to a solubilized composition, and in particular to improvement in solubilization of a carboxy-modified silicone in a water system in a large amount and in a stable manner.

### BACKGROUND OF THE INVENTION

A solubilization technique by which a water (solvent)-insoluble substance is transparently and uniformly dissolved has been widely used in the fields of cosmetic products, pharmaceuticals, foods, and the like. In general, a surfactant is used for solubilizing a sparingly soluble substance, and a concentration thereof higher than cmc allows a sparingly soluble substance to be incorporated in a micelle and to be dissolved, thereby being solubilized. Making a system be in such a solubilized state allows for provision of thermodynamic stability to a composition.

In particular, for cosmetic products, upon producing a water-based cosmetic such as a transparent lotion or a serum, an EO or PO addition type nonionic surfactant is added in an appropriate amount as a solubilizing agent for the purpose of solubilizing an oil agent such as a fragrance composed of ester, aldehyde, ketone, alcohol, ether, phenol, lactone, or the like in an aqueous solvent.

Meanwhile, in recent years, silicone oils, which have a high thermal stability and safety as well as various characteristics, and also have an excellent feeling after use, have frequently been used as oil agents for cosmetics. Above them, a carboxy-modified silicone oil in which a dimethylpolysiloxane chain of silicone oil is modified by a carboxyl group is expected to be used as a surfactant and the like because of the high chemical reactivity and adsorptive properties of a carboxyl group being a substituent, and is also known to be added to a hair cosmetic (Patent Document 1: International Publication No. WO 2004/091562 (Japanese Patent Application No. 2004-570862)).

Furthermore, as a modified silicone in which a different hydrophilic functional group is introduced, a polyether-modified silicone in which a dimethylpolysiloxane chain is modified by a polyoxyethylene group improves emulsion stability, and it is used for a cosmetic (Patent Document 2: Japanese Patent No. 3484300).

### DISCLOSURE OF THE INVENTION

### PROBLEM TO BE SOLVED BY THE INVENTION

In general, however, while the amount of an oil agent to be solubilized depends greatly on the structure and the amount of a solubilizing agent according to a substance to be solubilized as well as the HLB thereof, there has not been known yet a solubilizing agent capable of solubilizing in water a large amount of a carboxy-modified silicone which is very different from the conventional oil agent in terms of its structure and properties.

In addition, since a polyether-modified silicone is nonionic, particles thereof easily aggregate during solubilization, and there has been a problem with a feeling after use such as stickiness due to having a polyoxyethylene chain.

The present invention has been made in view of such problems of the prior art, and an object thereof is to provide a solubilized composition in which a carboxy-modified silicone is solubilized in a large amount and stably.

### MEANS TO SOLVE THE PROBLEM

In order to achieve the object, the present inventors and others have made intense studies, and as a result, have found that addition of a nonionic surfactant having a specific structure allows a carboxy-modified silicone to be solubilized in water in an extremely large amount to give a transparent to almost transparent aqueous solution excellent in stability, thereby leading to the completion of the present invention.

That is, a solubilized composition according to the present invention is characterized by consisting of:
(a) one or two or more nonionic surfactant(s), having a weighted average of the HLB value of 10 to 15, selected from polyoxyethylene alkyl ether type nonionic surfactant represented by the following formula (I) or (II):

   R¹-O-(CH₂CH₂O)ₐ-H (I)

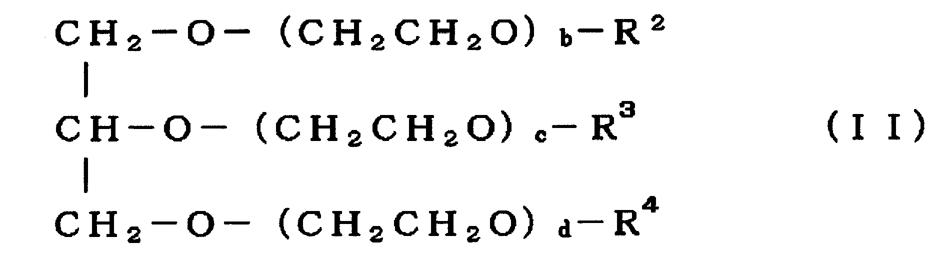

   wherein R¹ represents a branched chain alkyl group having 8 to 18 carbon atoms; a represents an integer satisfying 3 ≤ a ≤ 40; any one or more of R², R³ and R⁴ are each a branched chain alkyl group having 8 to 18 carbon atoms and the other(s) is a hydroxy group; and b, c and d each represent an integer satisfying 3 ≤ b + c + d ≤ 40,
(b)a carboxy-modified silicone and
(c) water, and
wherein the amount of (b) the carboxy-modified silicone is three times or less the weight of (a) the nonionic surfactant(s).

In addition, in the solubilized composition, it is preferable that a is an integer satisfying 8 ≤ a ≤ 30 and b, c and d are each an integer satisfying 8 ≤ b + c + d ≤ 30 in (a) the nonionic surfactant(s).

In addition, in the solubilized composition, it is preferable that (a) the nonionic surfactant(s) consists of one or two or more selected from the group consisting of POE isotridecyl ether, POE isostearyl ether, POE isocetyl ether, and POE glyceryl isostearate.

In addition, in the solubilized composition, it is preferable that (b) the carboxy-modified silicone is one in which a methyl group at one or more side chains or one terminal of polydimethylpolysiloxane is substituted with the following substituent:

-R⁵-COOH

wherein R⁵ is a straight-chain alkyl represented by -(CH₂)ₖ-, wherein k is an integer of 10 to 30.

In addition, the solubilized composition according to the present invention is characterized by consisting of the components (a) to (c) and (d) an alkyl-modified silicone represented by the following formula (V): 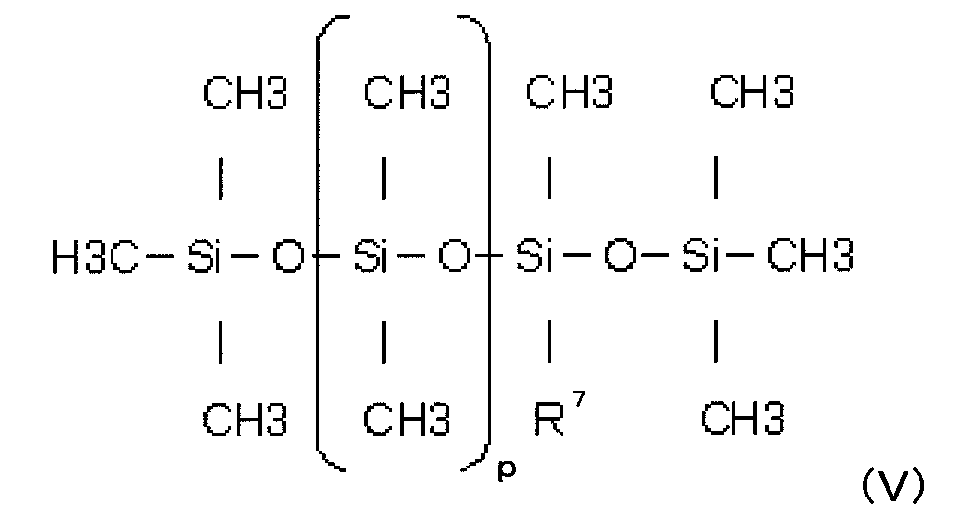 wherein R⁷ is an alkyl group having 1 to 12 carbon atoms and p is an integer of 0 to 2230.

In addition, the solubilized composition, the solubilized composition according to claim 5, **characterized in that** (d) the alkyl-modified silicone is dimethylpolysiloxane and/or caprylyl methicone

In addition, a cosmetic according to the present invention is characterized by comprising the solubilized composition.

### EFFECT OF THE INVENTION

According to the present invention, a solubilized composition can be obtained in which a sparingly soluble carboxy-modified silicone is solubilized in water in a large amount and stably. Further, according to the solubilized composition, a carboxy-modified silicone which has been conventionally resistant to addition to a water-based cosmetic, and also an alkyl-modified silicone can be added stably.

### DESCRIPTION OF THE PREFERRED EMBODIMENT

(a) The polyoxyethylene alkyl ether type nonionic surfactant used in the present invention is selected from those represented by the following formula (I) or (II):

R¹-O-(CH₂CH₂O)ₐ-H (I)

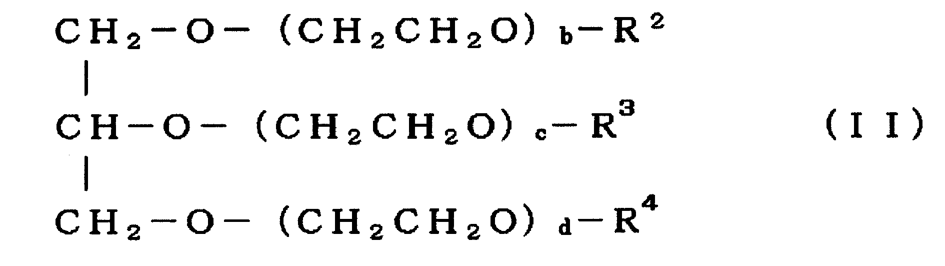

In the formulae, R¹ represents a branched chain alkyl group having 8 to 18 carbon atoms. Further, any one or more of R² to R⁴ are each a branched chain alkyl group having 8 to 18 carbon atoms, and the other R which is not an alkyl group is a hydroxy group. Specific examples of the branched chain alkyl group having 8 to 18 carbon atoms include an isotridecyl, isocetyl, and isostearyl groups.

In addition, a and b + c + d each denote an average number of moles added of ethyleneoxide (CH₂CH₂O) and are each an integer of 3 to 40, more preferably an integer of 8 to 30.

It is to be noted that in the formulae (I) and (II), the hydrophilic-lipophilic balance (HLB value) varies according to the average number of moles added of ethyleneoxide denoted by a and b + c + d and the chain lengths of alkyl groups represented by R¹ and R² to R⁴. Here, the HLB value is generally known as a value (hydrophilic-lipophilic balance) which represents the affinity of a surfactant for water and oil, and can be easily determined by a known calculation method such as Griffn's method.

In the solubilization of the carboxy-modified silicone in the present invention, one or a combination of two or more of the polyoxyethylene alkyl ether type nonionic surfactants is preferably used in which the average number of moles added of ethyleneoxide and the chain lengths of alkyl groups are within the numerical value ranges and the weighted average of the HLB value is 10 to 15. If the weighted average of the HLB value is less than 10, the lipophilicity is so high that the surfactant is resistant to dissolution in water in some cases, whereas if the weighted average exceeds 15, the hydrophilicity is so high that the solubilized composition is sticky in some cases. It is to be noted that if a surfactant having an HLB value less than 10 alone is used, a surfactant having a high HLB value is favorably simultaneously used to adjust the total HLB value.

That is, the present invention encompasses, in addition to the use of one or two or more of the nonionic surfactants having an HLB value of 10 to 15, the combination use of the nonionic surfactant having an HLB value less than 10 and another nonionic surfactant having the weighted average of the HLB value of 10 to 15.

In addition, the nonionic surfactant is preferably constituted by one or two or more polyoxyethylene alkyl ether type nonionic surfactants selected from in particular, POE isotridecyl ether, POE isostearyl ether, POE isocetyl ether, and POE glyceryl isostearate.

Examples of such a polyoxyethylene alkyl ether type nonionic surfactant include POE (8) isotridecyl ether [HLB13], POE (10) isostearyl ether [HLB11], POE (10) isocetyl ether [HLB10], POE (15) isocetyl ether [HLB13], POE (15) glyceryl isostearate [HLB12], and POE (20) glycerin triisostearate. Among them, POE (15) glyceryl isostearate [HLB8] is suitably used from the viewpoint of the structure of a branched chain, the molecular weight, and high solubilizing ability related to HLB.

The nonionic surfactant is easily dissolved in water to be a transparent aqueous solution. Further, a carboxy-modified silicone is added to this solution thereby to be solubilized, which makes it possible to obtain the solubilized composition of the present invention as a transparent to almost transparent aqueous solution. In this case, it is considered that a three-component system: nonionic surfactant/carboxy-modified silicone/water forms a bi-continuous phase.

(b) The carboxy-modified silicone that can be solubilized in the nonionic surfactant solution is a compound in which a functional group comprising a carboxyl group is introduced to a side chain and/or terminal of polydimethylpolysiloxane (straight silicone).

In particular, in the present invention, a carboxy-modified silicone in which a methyl group at one or more side chains or one terminal of polydimethylpolysiloxane is substituted with the following substituent is preferably used from the viewpoint of the amount solubilized:

-R⁵-COOH

In the formula, R⁵ is a straight alkyl represented by -(CH₂)ₖ-, wherein k is an integer of 10 to 30.

As a carboxy-modified silicone in which a methyl group at a side chain of polydimethylpolysiloxane is substituted (side chain type), one represented by the following formula (III) is preferable, and as a carboxy-modified silicone in which a methyl group at one terminal of polydimethylpolysiloxane is substituted (one terminal type), one represented by the following formula (IV) is preferable:

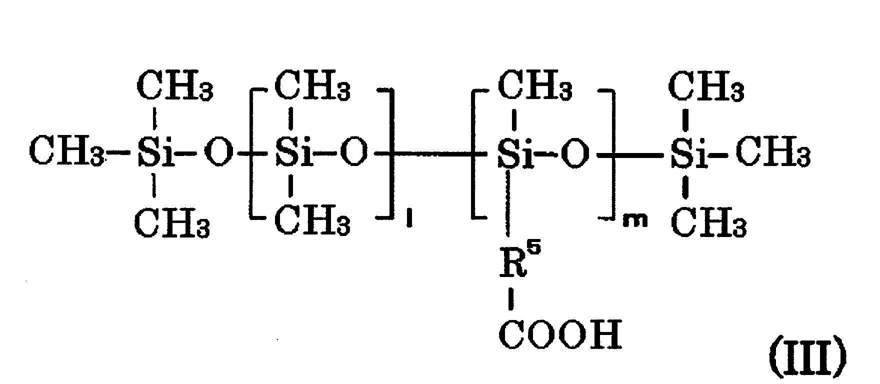

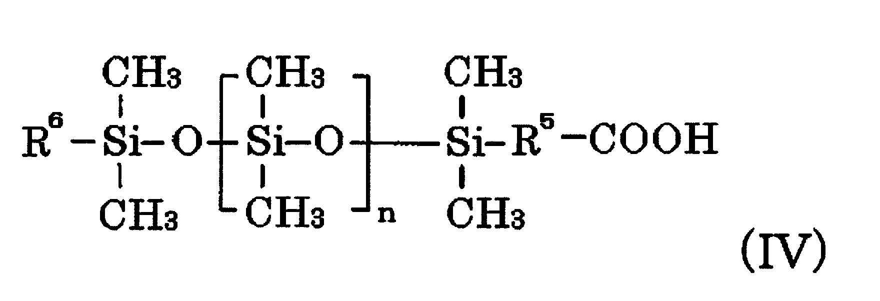

In the formulae (III) and (IV), R⁵ is a straight alkyl represented by -(CH₂)ₖ-, wherein k is an integer of 10 to 30.

R⁶ represents an alkyl group, an alkenyl group, an aryl group, an aralkyl group, or an alkylaryl group, each of which is optionally substituted with a halogen atom and has 18 or fewer carbon atoms.

In addition, 1 represents an integer of 30 to 600, m represents an integer of 1 to 20, and n represents an integer of 10 to 400.

Examples of the side chain type carboxy-modified silicone represented by the formula (III) include "X-22-3701E" produced by Shin-Etsu Chemical Co., Ltd., examples of the one terminal type carboxy-modified silicone represented by the formula (IV) include "X-22-3710" produced by the company, and these can be suitably used.

It is here considered that in the case of the carboxy-modified silicone, a smaller amount of the carboxyl group to be added makes the carboxy-modified silicone more hydrophobic, thereby changing the amount of the carboxy-modified silicone solubilized in an aqueous solution of a solubilizing agent. The amount of the carboxyl group to be added is not particularly limited in the present invention, but the carboxy-modified silicone with a functional group equivalent of a carboxyl group of 1000 to 5000 g/mol is preferable in terms of solubilization efficiency.

The solubilized composition according to the present invention includes the essential components, that is, (a) a nonionic surfactant having an HLB value in a specific range, (b) a carboxy-modified silicone, and (c) water, and can be prepared by appropriately mixing and dissolving the components (a) to (c) under heating.

The amount of each of the components added is as follows: the amount of (c) water is 60% or more by weight relative to the solubilized composition, and the amount of (b) the carboxy-modified silicone added is three times or less ((b)/(a)≤ 3/1), more preferably two times or less ((b)/(a)≤ 2/1) the weight of (a) the nonionic surfactant added.

If the amount of water added is too small, the nonionic surfactant is not sufficiently dissolved in the composition in some cases, whereas if the ratio of the carboxy-modified silicone to the nonionic surfactant added does not fall within the range, the separation of the system is caused, thereby failing to achieve sufficient solubilization in some cases.

Furthermore, (d) an alkyl-modified silicone having a specific structure can be also added to the solubilized composition according to the present invention in addition to the components, and the resultant mixture can also be solubilized. That is, the solubilized composition including the components (a) to (c) and (d) the alkyl-modified silicone having a specific structure is encompassed in the embodiments of the present invention. Usually, although an alkyl-modified silicone without a hydrophilic group is hardly alone dissolved in water or an aqueous nonionic surfactant solution, the solubilization of the alkyl-modified silicone is significantly facilitated in the solubilized composition of the present invention in which (b) the carboxy-modified silicone is dissolved.

The alkyl-modified silicone used as the component (d) of the present invention is a compound represented by the following formula (V). In the following formula, R⁷ is an alkyl group having 1 to 12 carbon atoms, and p is an integer of 0 to 2230.

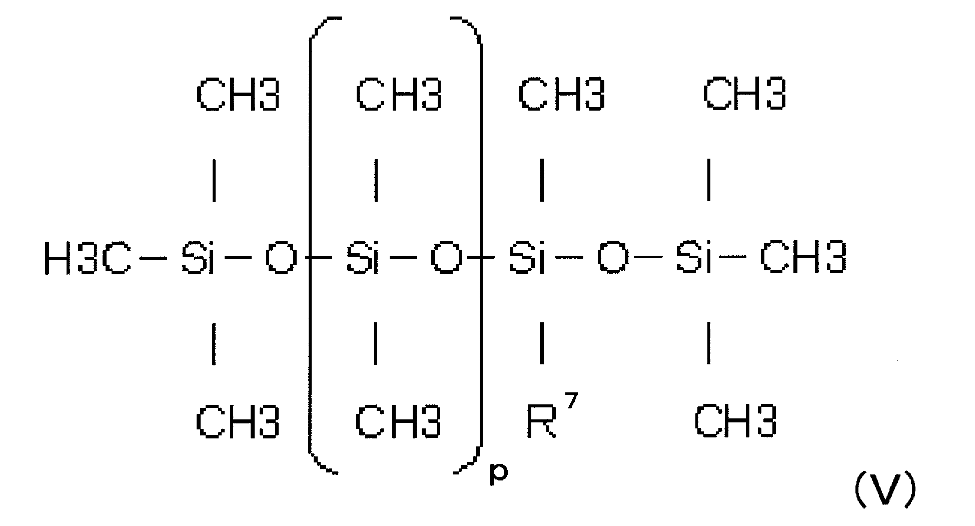

Among the alkyl-modified silicones represented by the formula (V), dimethylpolysiloxane or caprylyl methicone is particularly preferably used.

Examples of a commercial product of such an alkyl-modified silicone include "KF-96A-6T" and "KF-96-20cs" produced by Shin-Etsu Chemical Co., Ltd., "Wacker-Belsil (registered trademark) DM1 PLUS" produced by Wacker Chemie AG, and "SS-3408" produced by Dow Coming Toray Co., Ltd.

In the present invention, the amount of the alkyl-modified silicone added may be appropriately adjusted depending on the constituting components of the solubilized composition, but the alkyl-modified silicone is preferably added so that the addition ratio, ((a) nonionic surfactant + (b) carboxy-modified silicone):(d) alkyl-modified silicone, by weight, is in the range from 1:0.1 to 1:10. In particular, the alkyl-modified silicone exhibits excellent solubilizing properties by being combined with a one terminal type carboxy-modified silicone as a substance to be solubilized (component (b)).

In addition, the solubilized composition of the present invention can be combined with a component usually available for cosmetic products, quasi drugs, pharmaceuticals, or the like as long as the effect of the present invention is not impaired, to prepare a cosmetic. That is, the cosmetic according to the present invention comprises the solubilized composition.

Examples of the component capable of being combined with the solubilized composition of the present invention are given below. (1) Humectants: polyethyleneglycol, propylene glycol, dipropylene glycol, hexylene glycol, glycerin, diglycerin, xylitol, maltitol, maltose, D-mannitol and the like.

(2) Water-soluble polymers: plant-derived polymer such as gum arabic, carrageenan, pectin, agar, quince seed, starch and algae colloid (brown alga extract); microbially-derived polymer such as dextran and pullulan; animal-derived polymer such as collagen, casein and gelatin; starch-based polymer such as carboxymethyl starch and methylhydroxypropyl starch; alginic acid-based polymer such as alginate sodium; vinyl polymer such as carboxy vinyl polymer (e.g. CARBOPOL); polyoxyethylene polymer; polyoxyethylene/polyoxypropylene copolymer; acrylic polymer such as sodium polyacrylate and polyacrylamide; water-soluble inorganic polymer such as bentonite, magnesium aluminum silicate and laponite; and the like.

(3) Ultraviolet absorbers: benzoic acid type ultraviolet absorber such as para-aminobenzoic acid; anthranilic acid type ultraviolet absorber such as methyl anthranilate; salicylic acid type ultraviolet absorber such as octyl salicylate and phenyl salicylate; cinnamic acid type ultraviolet absorber such as isopropyl para-methoxycinnamate, octyl para-methoxycinnamate and mono 2-ethylhexanoate glyceryl dipara-methoxycinnamate; benzophenone type ultraviolet absorber such as 2,4-dihydroxybenzophenone, 2-hydroxy-4-methoxybenzophenone and 2-hydroxy-4-methoxybenzophenone-5-sulfonic acid; urocanic acid; 2-(2'-hydroxy-5'-methylphenyl) benzotriazole; 4-tert-butyl-4'-methoxybenzoylmethane; and the like.

(4) Sequestrants: sodium salt of edetic acid, sodium metaphosphate, phosphoric acid and the like.

(5) Antioxidants: ascorbic acid, alpha-tocopherol, dibutylhydroxytoluene, butylhydroxyanisol and the like.

(6) Drugs: vitamin such as vitamin A oil, retinal, retinol palmitate, inositol, pyridoxine hydrochloride, benzyl nicotinate, nicotinic-acid amide, dl-alpha-tocopherol nicotinate, ascorbic acid magnesium phosphate, ascorbic acid 2-glucoside, vitamin D2 (ergocalciferol), L-ascorbic acid dl-alpha-tocopherol phosphoric acid diester potassium salt, dl-alpha-tocopherol, dl-alpha-tocopherol acetate, pantothenic acid and biotin; hormone such as estradiol and ethinyl estradiol; anti-inflammatory agent such as allantoin and azulene; skin-lightening agent such as arbutin; astringent agent such as zinc oxide and tannin; algefacient such as L-menthol and camphor; sulfur; lysozyme chloride; pyridoxine hydrochloride; gamma-oryzanol; and the like.

(7) Various extracts: houttuynia extract, phellodendron amurense extract, melilot extract, lamium album extract, licorice (glycyrrhiza glabra) extract, peony root extract, saponaria officinalis extract, luffa cylindrica extract, cinchona succirubra bark extract, saxifraga sarmentosa extract, sophora angustifolia extract, nuphar japonicum extract, fennel extract, primula extract, rose extract, rehmannia chinensis root extract, lemon extract, lithospermum officinale extract, aloe extract, acous calamus root extract, eucalyptus globules extract, horsetail extract, sage extract, thyme extract, tea extract, alga extract, cucumber extract, clove extract, raspberry extract, melissa extract, panax ginseng root extract, carrot extract, horse chestnut extract, peach extract, peach leaf extract, mulberry extract, cornflower extract, hamamelis virginiana extract, silk extract and the like.

(8) Others: powders, pigments, pH adjuster, preservative (e.g. phenoxyethanol) and the like.

The various components can be used not only in a free state but also in an acid or base form, when possible, and an ester derivative thereof can also be used when having a carboxyl group. Further, if necessary, a surfactant other than the essential components can also be added to the solubilized composition of the present invention. Examples of such a surfactant include an anionic surfactant, a cationic surfactant, a nonionic surfactant, and an amphoteric surfactant that are generally added to a cosmetic or the like.

Since the solubilized composition of the present invention is excellent in stability, solubilizing ability, and feeling after use, it is particularly useful as a cosmetic product or a pharmaceutical, and it can be formed into a liquid formulation such as a lotion, an aftershave lotion, a serum, a body lotion, a hair tonic, a hair liquid, a hair restorer, and a fragrance. Further, the solubilized composition of the present invention can also be applied to other formulations as long as the effect of the present invention is not impaired. For example, the solubilized composition of the present invention can also be formed into a gel formulation by using a known thickening agent or the like, a sheet formulation by being impregnated in a nonwoven cloth or the like, or a spray, an aerosol, or a roll-on type formulation by a known method.

Further, a cosmetic produced (used) by diluting the solubilized composition component of the present invention is encompassed herein.

### EXAMPLES

Hereinafter, the present invention will be described with reference to specific examples, but it is not to be limited thereto. The amount added is expressed in % by weight, unless otherwise specified. It is to be noted that a test method used below is as follows.

### (Test Method)

In accordance with the formulation shown in Table 1 below, a nonionic surfactant, a carboxy-modified silicone and water were dissolved and mixed at 70°C to obtain a sample of each of Examples and Comparative Examples. The solubilization of each sample was evaluated by measuring transparency as follows.

### Measurement Method

The transparency of each sample was confirmed by measuring an L value. The L value was measured as the transparency when assuming the value of distilled water serving as a control as 100 by using a spectrophotometer (UV-160) manufactured by Shimadzu Corporation. The evaluation criteria for solubilization are as follows.

### Evaluation Criteria

⊚ : The L value (transparency) is 95 or more (sample is transparent and carboxy-modified silicone is completely solubilized).
○ : The L value (transparency) is 85 to less than 95 (sample is almost transparent and carboxy-modified silicone is almost completely solubilized).
× : The L value (transparency) is less than 85 (sample is cloudy to translucent and carboxy-modified silicone is hardly solubilized).

Unmeasurable: A separated product is observed.

**TABLE 1**

| | Example | | | | | Comparative Example | | | |
|---|---|---|---|---|---|---|---|---|---|
| Formulation | 1 | 2 | 3 | 4 | 5 | 1 | 2 | 3 | 4 |
| (Nonionic surfactant) | | | | | | | | | |
| POE (8) isotridecyl ether | 0.5 | - | - | 0.5 | - | - | - | - | - |
| POE (10) isostearyl ether | - | 0. 5 | - | - | - | - | - | - | - |
| POE (10) isocetyl ether | - | - | 0.5 | - | - | - | - | - | - |
| POE (15) glyceryl isostearate | - | - | - | - | 0.5 | - | - | - | - |
| POE (5) stearyl ether | - | - | - | - | - | 0. 5 | - | - | - |
| POE (10) stearyl ether | - | - | - | - | - | - | 0.5 | - | - |
| POE (15) stearyl ether | - | - | - | - | - | - | - | 0. 5 | - |
| POE (40) hydrogenated castor oil | - | - | - | - | - | - | - | - | 0. 5 |

| (Carboxy-modified silicone) | | | | | | | | | |
|---|---|---|---|---|---|---|---|---|---|
| Side chain type (X-22-3701 E) | 1 | 1 | 1 | - | - | 1 | 1 | 1 | 1 |
| One terminal type (X-22-371 0) | - | - | - | 1 | 1 | - | - | - | - |
| Water | Balance | Balance | Balance | Balance | Balance | Balance | Balance | Balance | Balance |
| Evaluation for solubilization | ⊚ | ⊚ | ⊚ | ⊚ | ⊚ | × | × | × | × |

As shown in Table 1, the side chain type and one terminal type carboxy-modified silicones were favorably solubilized to be transparent to almost transparent liquid compositions in Examples 1 to 5 each using a nonionic surfactant with a branched alkyl chain.

On the other hand, all the carboxy-modified silicones were not solubilized and the compositions became cloudy or were completely separated into two layers in Comparative Examples 1 to 4 each using a straight alkyl type nonionic surfactant.

It has been revealed from the foregoing results that, in the present invention, addition of the nonionic surfactant having a specific structure allows obtaining a transparent to almost transparent solubilized composition with a carboxy-modified silicone dissolved therein.

In addition, when the nonionic surfactant having a specific structure was subjected to a more detailed solubilization test, POE (15) glyceryl isostearate was particularly suitable.

Then, prepared were compositions each containing nonionic surfactant POE (8) isotridecyl ether [HLB13] and the side chain or one terminal type carboxy-modified silicone "X-22-3701E" or "X-22-3710" produced by Shin-Etsu Chemical Co., Ltd. in an addition ratio (% by weight) shown in the following Tables 2 and 3, and the solubilized state of the composition of each of Test Examples was evaluated according to the criteria.

The test results when using the side chain type carboxy-modified silicone are shown in Table 2, and the test results when using the one terminal type carboxy-modified silicone are shown in Table 3.

**TABLE 2**

| | Test Example | | | | | | | | |
|---|---|---|---|---|---|---|---|---|---|
| Formulation | 1 | 2 | 3 | 4 | 5 | 6 | 7 | 8 | 9 |
| POE (8) isotridecyl ether | 20 | 18 | 16 | 14 | 12 | 10 | 8 | 6 | 4 |
| Side chain type (X-22-3701E) | - | 2 | 4 | 6 | 8 | 10 | 12 | 14 | 16 |
| Water | Balance | Balance | Balance | Balance | Balance | Balance | Balance | Balance | Balance |
| Evaluation for solubilization | ⊚ | ⊚ | ⊚ | ⊚ | ⊚ | ⊚ | ⊚ | ○ | × |

**TABLE 3**

| | Test Example | | | | | | | | |
|---|---|---|---|---|---|---|---|---|---|
| Formulation | 10 | 11 | 12 | 13 | 14 | 15 | 16 | 17 | 18 |
| POE (8) isotridecyl ether | 20 | 18 | 16 | 14 | 12 | 10 | 8 | 6 | 4 |
| One terminal type (X-22-3701 E) | - | 2 | 4 | 6 | 8 | 10 | 12 | 14 | 16 |
| Water | Balance | Balance | Balance | Balance | Balance | Balance | Balance | Balance | Balance |
| Evaluation for solubilization | ⊚ | ⊚ | ⊚ | ⊚ | ⊚ | ⊚ | ⊚ | ○ | × |

As shown in Tables 2 and 3, POE (8) isotridecyl ether had a high solubilization power of the carboxy-modified silicone and favorably solubilized the carboxy-modified silicone in a weight three times or less the weight of itself (Test Examples 1 to 8 and 10 to 17), in particular two times or less the weight of itself (Test Examples 1 to 7 and 10 to 16). On the other hand, if the weight of the carboxy-modified silicone exceeded three times that of the nonionic surfactant, a transparent solubilized composition was not obtained (Test Examples 9 and 18).

Furthermore, when other nonionic surfactants applicable to the present invention were also subjected to the test, a tendency similar to the results shown in Tables 2 and 3 was observed.

Therefore, the amount of the carboxy-modified silicone added is three times or less, more preferably two times or less the weight of the nonionic surfactant having a specific structure.

In addition, in the case of combining polyoxyethylene alkyl ether type nonionic surfactants each having a different HLB, the solubilized state of a composition obtained by adding the combined surfactants in the following addition ratio (% by weight) was evaluated as described above. Further, the weighted average of POE (20) glyceryl isostearate and POE (20) glyceryl triisostearate was calculated and the solubilized state based on the average was also evaluated. The results are shown in Table 4.

**TABLE 4**

| | Test Example | | | | | | | | | |
|---|---|---|---|---|---|---|---|---|---|---|
| Formulation | 19 | 20 | 21 | 22 | 23 | 24 | 25 | 26 | 27 | 28 |
| POE (20) glyceryl isostearate [HLB 13] | | | | | | | | | | |
| | 8 | 7 | 6 | 5 | 4 | 3 | 2 | 1 | 1 | 0 |
| POE (20) glyceryl triisostearate [HLB 8] | | | | | | | | | | |
| | 2 | 1.75 | 1.5 | 1.25 | 1 | 0.75 | 0.5 | 0.25 | 4 | 10 |
| One terminal type (X-22-3701E) | 1 | 2 | 3 | 4 | 5 | 6 | 7 | 8 | 5 | 10 |
| Water | Balance | Balance | Balance | Balance | Balance | Balance | Balance | Balance | Balance | Balance |
| Weighted average of HLB value | 12 | 12 | 12 | 12 | 12 | 12 | 12 | 12 | 9 | 8 |
| Evaluation for solubilization | ⊚ | ⊚ | ⊚ | ⊚ | ⊚ | ⊚ | ○ | × | × | × |

The composition obtained by combining the different polyoxyethylene alkyl ether type nonionic surfactants also favorably solubilized the carboxy-modified silicone in a weight three times or less (Test Examples 19 to 25) the total weight of the nonionic surfactants, and in particular was rated highly in the carboxy-modified silicone in a weight two times or less (Test Examples 19 to 24). On the other hand, when the weight of the carboxy-modified silicone was more than three times the weight of the nonionic surfactant, the carboxy-modified silicone was not solubilized (Test Example 26).

In addition, with regard to the HLB value of the nonionic surfactant, in Test Examples 27 and 28 in which the weighted average of the HLB value of the nonionic surfactant was less than 10, the nonionic surfactant was not solubilized sufficiently although the weight ratio relation was satisfied. A more detailed study of the range of the weighted average of the HLB value has made it clear that when the weighted average is 10 to 15, a transparent to almost transparent solubilized composition is obtained.

In addition, a further attempt was made to solubilize alkyl-modified silicones (dimethylpolysiloxane) in solubilized compositions each having a different ratio of the nonionic surfactant POE (8) isotridecyl ether and the side chain type or one terminal type carboxy-modified silicone ("X-22-3701E" or "X-22-3710" produced by Shin-Etsu Chemical Co., Ltd.) added. The test results are shown in Table 5.

### (Test Method)

The nonionic surfactant, the carboxy-modified silicone, and water were dissolved and mixed at 70°C, dimethylpolysiloxane (KF-96A-6cs produced by Shin-Etsu Chemical Co., Ltd.) was added thereto in a variety of concentrations (1 to 6% by weight relative to the composition), and whether the resultant compositions could be solubilized or not was evaluated according to the criteria.

**TABLE 5**

| | Test Example | | | | | | | | | | | |
|---|---|---|---|---|---|---|---|---|---|---|---|---|
| Formulation | 29 | 30 | 31 | 32 | 33 | 34 | 35 | 36 | 37 | 38 | 39 | 40 |
| POE (8) isotridecyl ether | 10 | 8 | 6 | 5 | 4 | 2 | 10 | 8 | 6 | 5 | 4 | 2 |
| Side chain type (X-22-3701 E) | - | 2 | 4 | 5 | 6 | 8 | - | - | - | - | - | - |
| One terminal type (X-22-3710) - | | - | - | - | - | - | - | 2 | 4 | 5 | 6 | 8 |
| Water | Balance | Balance | Balance | Balance | Balance | Balance | Balance | Balance | Balance | Balance | Balance | Balance |

| (Evaluation for solubilization) | | | | | | | | | | | | |
|---|---|---|---|---|---|---|---|---|---|---|---|---|
| Dimethylpolysiloxane 1% | × | ⊚ | ⊚ | ⊚ | ○ | × | × | × | ⊚ | ⊚ | ⊚ | × |
| Dimethylpolysiloxane 2% | × | × | ○ | ○ | ○ | × | × | × | ⊚ | ⊚ | ⊚ | × |
| Dimethylpolysiloxane 3% | × | × | × | × | × | × | × | × | ○ | ○ | ○ | × |
| Dimethylpolysiloxane 4% | × | × | × | × | × | × | × | × | × | ○ | ○ | × |
| Dimethylpolysiloxane 5% | × | × | × | × | × | × | × | × | × | × | ○ | × |
| Dimethylpolysiloxane 6% | × | × | × | × | × | × | × | × | × | × | × | × |

As shown in Table 5, dimethylpolysiloxane, which is a sparingly soluble component, was not alone solubilized in POE (8) isotridecyl ether (Test Examples 29 and 35), but it was solubilized therein with a system having the carboxy-modified silicone solubilized therein (Test Examples 30 to 34 and 36 to 40) depending on the concentration of the carboxy-modified silicone added. In particular, in Test Example 39 in which the amount of the one terminal type carboxy-modified silicone added was 1.5 times that of the nonionic surfactant in terms of weight, the high concentration of dimethylpolysiloxane was solubilized.

It is obvious from the foregoing results that the solubilized composition of the present invention also allows dimethylpolysiloxane to be solubilized.

Furthermore, an attempt was also made to solubilize other commercial alkyl-modified silicones having a different molecular weight in a composition with nonionic surfactant/carboxy-modified silicone/water. The results are shown in Table 6.

### (Test Method)

The nonionic surfactant, the carboxy-modified silicone, water and each of alkyl-modified silicones were dissolved and mixed at 70°C, then cooled to obtain a composition, and whether the composition could be solubilized or not was evaluated according to the method.

**TABLE 6**

| Example | 41 | 42 | 43 | 44 | 45 | 46 | 47 | 48 | 49 | 50 | 51 | 52 |
|---|---|---|---|---|---|---|---|---|---|---|---|---|
| POE (8) isotridecyl ether | 5 | 5 | 5 | 5 | 5 | 5 | | | | | | |
| POE (15) glyceryl isostearate | | | | | | | 6 | 6 | 6 | 6 | 6 | 6 |
| Side chain type (X-22-3701 E) | 5 | 5 | 5 | | | | 4 | 4 | 4 | | | |
| One terminal type (X-22-3710) | | | | 5 | 5 | 5 | | | | 4 | 4 | 4 |
| Octamethyltrisiloxane (Wacker-Belsil(R) DM1 PLUS, Wacker Chemie AG) | 3 | | | 4 | | | 3 | | | 2 | | |
| Dimethylpolysiloxane (KF-96A-6T, Shin-Etsu Chemical Co., Ltd.)) | | 2 | | | 3 | | | 1 | | | 4 | |
| Dimethylpolysiloxane (KF-96A-20cs, Shin-Etsu Chemical Co., Ltd.)) | | | 1 | | | 1 | | | | | | 2 |
| Heptamethyloctyltrisiloxane (SS-3408, Dow Corning Toray Co.,Ltd.) | | 1 | | | | 1 | | | 2 | 1 | | |
| Water | Balance | Balance | Balance | Balance | Balance | Balance | Balance | Balance | Balance | Balance | Balance | Balance |
| | | | | | | | | | | | | |
| (Evaluation for Solubilization) | ⊚ | ⊚ | ⊚ | ⊚ | ⊚ | ⊚ | ⊚ | ⊚ | ⊚ | ⊚ | ⊚ | ⊚ |

As shown in Table 6, it was confirmed that the alkyl-modified silicone having a different molecular weight was solubilized with the nonionic surfactant and the carboxy-modified silicone. Further, the combination of the different alkyl-modified silicones was also enabled to be solubilized (Test Examples 42, 46, and 50).

It is obvious from the foregoing results that the solubilized composition of the present invention also allows the alkyl-modified silicone to be solubilized.

In addition, it has been found from the results of an additional study that when the weight ratio of (nonionic surfactant + carboxy-modified silicone):alkyl-modified silicone is 1:0.1 to 1:10, a stable and transparent solubilized composition can be obtained.

Hereinafter, formulation examples of the present invention will be described with reference to specific examples, but the present invention is not to be limited thereto.

### Formulation Example 1: Lotion

| (Component) | (% by weight) |
|---|---|
| POE (10) isocetyl ether | 4 |
| Carboxy-modified silicone (X-22-3701E, Shin-Etsu Chemical Co., Ltd.) | 6 |
| Sodium citrate | 0.4 |
| Phenoxyethanol | 0.3 |
| Water | Balance |
| (Production method) | |

POE (10) isocetyl ether, carboxy-modified silicone and water were mixed under high temperature (70 degrees C), and then the mixture was cooled.

### Formulation Example 2: Lotion

| (Component) | (% by weight) |
|---|---|
| POE (15) glyceryl isostearate | 0.5 |
| Carboxy-modified silicone (X-22-3710, Shin-Etsu Chemical Co., Ltd.) | 1 |
| Sodium citrate | 0.2 |
| Phenoxyethanol | 0.5 |
| Water | Balance |

### (Production method)

POE (15) glyceryl isostearate, carboxy-modified silicone and water were mixed under high temperature (70 degrees C), and then the mixture was cooled.

### Formulation Example 3: Lotion

| (Component) | (% by weight) |
|---|---|
| POE (20) glyceryl isostearate | 0.5 |
| POE (20) glyceryl triisostearate | 0.1 |
| Carboxy-modified silicone (X-22-3710 Shin-Etsu Chemical Co., Ltd.) | 0.4 |
| Dimethylpolysiloxane (KF-96A-6T, Shin-Etsu Chemical Co., Ltd.) | 0.2 |
| Sodium citrate | 0.2 |
| Phenoxyethanol | 0.5 |
| Water | Balance |

### (Production method)

Nonionic surfactants, carboxy-modified silicone, dimethylpolysiloxane and water were mixed under high temperature (70 degrees C), and then the mixture was cooled.

## Claims

1. A solubilized composition consisting of:
(a) one or two or more nonionic surfactant(s), having a weighted average of the HLB value of 10 to 15, selected from polyoxyethylene alkyl ether type nonionic surfactant represented by the following formula (I) or (II):
R¹-O-(CH₂CH₂O)ₐ-H (I) 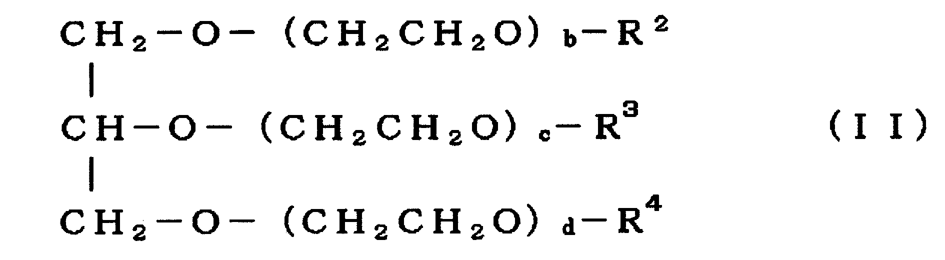
wherein R¹ represents a branched chain alkyl group having 8 to 18 carbon atoms; a represents an integer satisfying 3 ≤a ≤ 40; any one or more of R², R³ and R⁴ are each a branched chain alkyl group having 8 to 18 carbon atoms and the other(s) is a hydroxy group; and b, c and d each represent an integer satisfying 3 ≤ b + c + d ≤ 40,
(b) a carboxy-modified silicone, and
(c) water, and
wherein the amount of (b) the carboxy-modified silicone is three times or less the weight of (a) the nonionic surfactant(s).

2. The solubilized composition according to claim 1, wherein a is an integer satisfying 8 ≤a ≤ 30 and b, c and d are each an integer satisfying 8 ≤ b + c + d ≤ 30 in (a) the nonionic surfactant(s).

3. The solubilized composition according to claim 1 or 2, wherein (a) the nonionic surfactant(s) consists of one or two or more selected from the group consisting of POE isotridecyl ether, POE isostearyl ether, POE isocetyl ether and POE glyceryl isostearate.

4. The solubilized composition according to any of claims 1 to 3, wherein (b) the carboxy-modified silicone is one in which a methyl group at one or more side chains or one terminal of polydimethylpolysiloxane is substituted with the following substituent:
-R⁵-COOH
wherein R⁵ is a straight-chain alkyl represented by -(CH₂)ₖ-, wherein k is an integer of 10 to 30.

5. A solubilized composition consisting of the components (a) to (c) and
(d) an alkyl-modified silicone represented by the following formula (V): 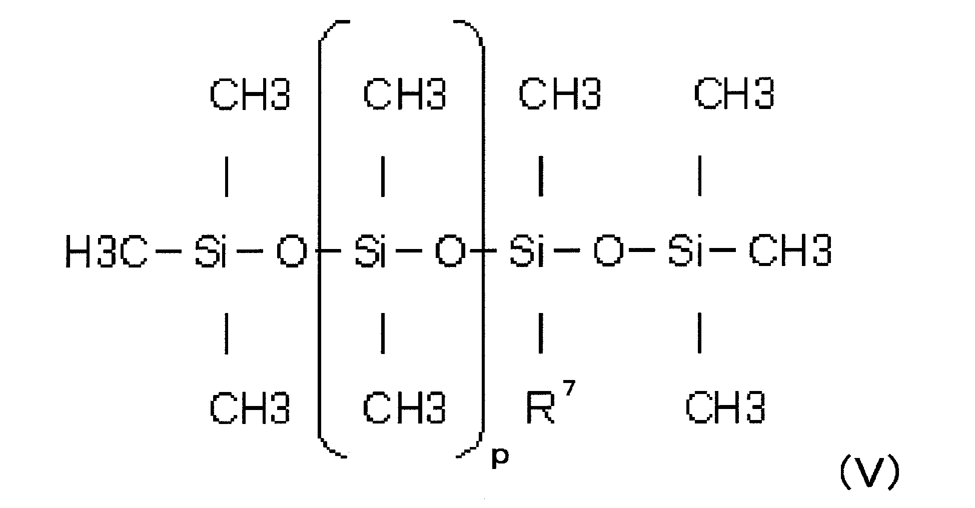 wherein R⁷ is an alkyl group having 1 to 12 carbon atoms; and p is an integer of 0 to 2230.

6. The solubilized composition according to claim 5, wherein (d) the alkyl-modified silicone is dimethylpolysiloxane and/or caprylyl methicone.

7. A cosmetic comprising the solubilized composition of any of claims 1 to 6.
